# EUROPEAN PATENT SPECIFICATION

(11) **EP 4 679 451 B1**
(45) Date of publication and mention of the grant of the patent: **25.03.2026**
(21) Application number: 24188258.8
(22) Date of filing: 12.07.2024
(51) Int. Cl.: G16H 50/20, G16H 50/70

(54) **COMPUTER SYSTEM AND METHOD FOR SUPPORTING MEDICAL DIAGNOSIS OF A HUMAN WITH SYMPTOMS ASSOCIATED WITH THE HUMAN'S MEDICAL CONDITION**
COMPUTERSYSTEM UND VERFAHREN ZUR UNTERSTÜTZUNG DER MEDIZINISCHEN DIAGNOSE EINES MENSCHEN MIT SYMPTOMEN IM ZUSAMMENHANG MIT DEM MEDIZINISCHEN ZUSTAND DES MENSCHEN
SYSTÈME INFORMATIQUE ET PROCÉDÉ POUR PRENDRE EN CHARGE LE DIAGNOSTIC MÉDICAL D'UN ÊTRE HUMAIN AVEC DES SYMPTÔMES ASSOCIÉS À L'ÉTAT MÉDICAL DU ÊTRE HUMAIN

(43) Date of publication of application: 14.01.2026
(73) Proprietor: Ada Health GmbH, 10179 Berlin (DE)
(72) Inventor: Hartenstein, Vera, 10179 Berlin (DE); Muscoloni, Alessandro, 10179 Berlin (DE); Stroux, Lisa, 10179 Berlin (DE); Köhler, Sebastian, 10179 Berlin (DE); Morelli, Valerio, 10179 Berlin (DE); Hoffmann, Henry, 10179 Berlin (DE); Engerer, Peter, 10179 Berlin (DE)
(74) Representative: Fleuchaus & Gallo Partnerschaft mbB

(56) References cited:
- CN-A- 117 542 509
- CN-A- 117 690 604
- HENGGUAN HUANG ET AL: "Verbalized Probabilistic Graphical Modeling with Large Language Models", ARXIV.ORG, CORNELL UNIVERSITY LIBRARY, 201 OLIN LIBRARY CORNELL UNIVERSITY ITHACA, NY 14853, 8 June 2024 (2024-06-08), XP091783276
- ANONYMOUS: "Dx-LLM: Two-layer Retrieval-Augmented Multilingual Diagnosis System", 2 July 2024 (2024-07-02), XP093319249, Retrieved from the Internet <URL:https://openreview.net/pdf?id=I7KZoCV50T> [retrieved on 20250926]

## Description

### Technical Field

The present invention generally relates to method and system for diagnosis support, and in particular, relates to computer-implemented method, computer program product and system for supporting medical diagnosis of a human with symptoms associated with the human's medical condition.

### Background

Symptom checker applications are used to support users in the medical diagnosis of a human with symptoms associated with the human's medical condition. An example of a prior art symptom checker is the Ada symptom checker application (Version 3.54.0) available at https://ada.com/de/app/.

Such prior art symptom checker applications are herein referred to as primary diagnosis models (PDM) and are often based on probabilistic graphical models (PGM): The PDM performs a guided assessment process in which the user is asked a plurality of questions (e.g., in the order of 20 questions) to collect evidences (i.e., symptoms and risk factors). This guided evidence gathering process is also referred to as question-flow. As output, the PDM provides a list of conditions and their posteriors, i.e., their probabilities given the evidences collected during the question-flow. Some conditions may be quite likely, some less likely, and some impossible. Typically, not the entire list of possible condition candidates is shown to the user but only a subset (e.g., up to five).

As symptom checkers are often used by users who are the patients themselves, a high accuracy is desirable with regard to potential identified conditions based on the collected evidence. The accuracy of prior art symptom checkers has room for improvement in this regard. The paper "Dx-LLM: Two-layer Retrieval-Augmented Multilingual Diagnosis System", 2 July 2024 (https://openreview.net/pdf?id=I7KZoCV50T), proposes a two-layer Retrieval-Augmented Multilingual Diagnosis System by leveraging retrieval-augmented large language models (RA-LLMs) to construct a disease-symptom knowledge graph from an external dataset of disease symptom descriptions for initial disease filtering to identify potential candidate diseases based on patient symptoms. Subsequently, in a second layer, language understanding and generation capabilities of LLMs are used to re-rank these candidates, thereby producing refined diagnostic outcomes. However, while Retrieval-Augmented Generation reduces hallucinations or confabulations compared with pure LLM generation, it can still produce erroneous or fabricated outputs - particularly in medical contexts.

### Summary

Hence, there is a need for medical diagnosis support applications with improved accuracy of identifying the potential medical condition(s) of the human based on the collected evidences. This technical problem is solved by the herein disclosed approach of combining a known PGM symptom checker with symptom assessment based on a Large Language Model (LLM), wherein the LLM is actually used as a classifier to further refine the results of the PGM. The herein disclosed approach can be implemented as a computer-implemented method, a computer program product and a computer system as described by the independent claims. Thereby, the LLM is provided with the user's evidences (symptoms and risk factors) collected in the questions-flow of the PGM. However, the LLM does not only get the evidences but also a list with possible conditions (condition list) from the PGM. This list may be ordered in descending order with respect to the probability that the user has a specific condition (i.e., it may start with the most probable condition according to the PGM). Furthermore, the condition list is used to constrain the output of the LLM which suppresses hallucinations of the LLM since it has to pick its outputs from said condition list, while the LLM is tasked to retry if its output fails to limit its suggestions to conditions from said condition list. In other words, the prompt is reapplied to the LLM until the generated output is in conformance with said suggestions (i.e., the classes defined by the one or more relevant primary causing conditions in the prompt), or a predefined abort criterion is met. This provides a safe-guard that the LLM is neither making up conditions which do not exist nor suggesting conditions which have been deemed impossible by the PGM which would take its application for medical diagnosis support at risk. The proposed approach can therefore be described as "safe prompting integrating constraints and expertise" (SPICE).

In one embodiment, a computer-implemented method is provided for supporting medical diagnosis of a human with symptoms associated to the human's medical condition. Thereby, the human user interacts with a primary diagnosis model (PDM) of a computer system. During such interaction, the PDM obtains medical state indicators of the user which are used as inputs of the PDM. The medical state indicators include the symptoms of said human. For example, the medical state indicators can be collected by the PDM in a dynamic question-flow process where the user responds to respective questions generated by the PDM. For example, the PDM may pose questions to the human user via a respective user interface (e.g., via graphical UI, a voice-controlled UI or a combination thereof). Examples for collecting symptoms can be the questions: "Do you have a headache?" or "Is your knee itchy?". An example for risk factors is the question: "Did you travel to sub-Saharan Africa recently?". Using a PGM (PDM) and information-gain for generating the next best question instead of prompting an LLM (SDM) to come up with a good question has the benefit of consuming less computational resources and lower latency.

Based on the human's answers to such questions as inputs, the PDM determines from a set of predefined medical conditions one or more possible causing conditions from the PDM (thereafter referred to as primary causing conditions) with associated primary causing condition probabilities for said human as an output. Thereby, a particular possible primary causing condition is a relevant primary causing condition that is associated with a relevant particular primary causing condition probability if the associated primary causing condition probability is above a predefined threshold or belongs to a predefined number (count) of highest ranking primary causing conditions. In other words, possible primary causing conditions associated with relevant particular primary causing condition probabilities are considered to be relevant primary causing conditions.

The PDM has a defined output space (e.g., an ontology) and is deterministic in that the same input always yields the same output. Possible implementations of the PDM include: a probabilistic graphical model (PGM), a decision tree, a support vector machine, a k-nearest neighbors classifier algorithm, or a logistic regression. The one or more medical state indicators may be obtained by several possible implementations including a PGM which uses information gain for choosing an appropriate next question in a questionnaire process applied to the human to obtain the one or more medical state indicators.

The PDM delivers probabilities for conditions (e.g., "there is: a 25,4% chance for having Chronic granulomatous disease, a 14,0% chance ...") but depends very much on precise information provided by the human user.

The obtained medical state indicators and one or more relevant primary causing conditions are combined into a prompt which is then provided as input to at least one (pre-trained) Large Language Model (LLM). The prompt may include a large list of relevant primary causing conditions (e.g., the top 100). The LLM is used as a secondary diagnosis model (SDM). The LLM is prompted to perform the role of a medical practitioner. That is, the SDM is instructed to generate an output of one or more possible secondary causing conditions with associated secondary causing condition probabilities for the obtained medical state indicators and in conformance with classes defined by the one or more relevant primary causing conditions provided as input to the SDM in the same prompt. In other words, the LLM also determines possible causing conditions with respective causing condition probabilities. While it is known that LLMs tend to hallucinate, which in a medical diagnosis system can be detrimental for the human user if the system hallucinates causing conditions for which no scientific basis exists, the generative outputs of the LLM can bring additional aspects into the diagnosis support by re-ranking the importance of the relevant causing conditions with respect to the PDM. This re-ranking can occur for several reasons including that LLMs can work with structured and unstructured data and can handle "fuzzy" user inputs like a vague description of the problem by the human user. It has a relatively "complete understanding" of existing conditions (e.g., obtained by training on internet scale data, including relevant medical data) and may even provide multimodal capabilities allowing to ingest data beyond simple text strings and free-text data, such as images, audio or video data. It is to be noted that those evidences (free-text and multimodal data) might not be collected by the PDM but could be directly received as user inputs being passed to the SDM.

The hallucination problem of LLMs is addressed by generating the SDM output of one or more possible secondary causing conditions with associated secondary causing condition probabilities in conformance with classes defined by the one or more relevant primary causing conditions in the prompt. In other words, the LLM is used like a classifier. As the PDM is a deterministic system which provides its output based on medical knowledge embedded in its data structures (e.g., ontology), the PDM outputs can be used like a guardrail function to eliminate hallucinations from the SDM output. Guardrails in the context of Large Language Models (LLMs) are mechanisms designed to ensure that the outputs of these models conform with defined rules, i.e., they are at least semantically correct and factually accurate. Their implementation is advantageous for achieving reliable and consistent outputs from LLMs. This is particularly relevant in the medical field where precision and regulatory compliance cannot be compromised. When using the LLM without such guardrail function, frequently hallucinations come up with plausible sounding conditions that do not even exist, such as "carpal tunnel syndrome in the ankle" while it is exclusively occurring in the wrist.

A further advantage of using a combination of an LLM with a PDM is an improved explainability of the results compared to an LLM as stand-alone solution. This is an important aspect in medical diagnosis. A reliable diagnosis of the human's medical condition should be explainable which is largely achieved by preserving the PDM's reasoning explanation regarding how the algorithm came to the solution, and how it was derived from the human's medical state indicators.

In the prior art, retrieval-augmented generation (RAG) describes a technique that is used to provide relevant documents to an LLM as an input. In the context of medical diagnosis, a relevant corpus for RAG could be medical literature, such as medical books, scientific papers, and medical forums. RAG usually works as follows: A user writes a query. The query is transformed into an embedding, and in a vector database the relevant document(s) are retrieved. There may be a re-ranking of the documents to filter for the most relevant ones. The documents are provided to the LLM in the prompt, and it can make use of them for answering a user query. In contrast, the herein disclosed method does not condition LLM generation on retrieved documents from respective medical literature, but rather on a list of candidate conditions. The candidate conditions are not produced by textual or semantic similarity, but by their likelihood according to the PDM output. This approach guarantees that there is a causal relationship between the evidence and the candidate conditions. By avoiding putting potentially large numbers of irrelevant tokens into the prompt (as it can be the case in RAG when long documents such as scientific papers are used in the corpus), the herein disclosed method uses a limited amount of tokens according to the relevant primary causing condition list, and thus is also favorable in terms of latency and computational cost with respect to RAG.

In general, LLMs are generative artificial intelligence models, i.e., they are used to generate next tokens (e.g., answer an open question). LLMs have been envisioned to be applied as symptom checkers or clinical decision support systems. However, hallucinations of LLMs may constitute a medical risk for patient users which complicates the application of LLMs in the medical field. In such prior art systems, the LLM is instructed to ask the user medical questions while being empathetic and truthful. After some back-and-forth the LLM comes up with a diagnosis based on the collected information. This type of "LLM-only" symptom checker is frequently enhanced by one of the following:
- The LLM is fine-tuned on medical data

- The LLM uses RAG to find relevant documents it can use for reasoning and/or for providing the information sources to the user (e.g., it is Parkinson's, based on paper XYZ, et al. (20XX)).

In the herein disclosed approach, the LLM is rather used as "classifier" by constraining the output to the PDM's space of relevant primary causing conditions. If the LLM output does not comply with the PDM classes, its output is not accepted, and the algorithm is run again. By avoiding the generation of irrelevant tokens, also latency and computational cost are minimal.

In one embodiment, the prompt provides one or more relevant primary causing conditions to the secondary diagnosis model in descending order of the associated relevant primary causing condition probabilities. Thereby, it has been found that using such a ranked list in the prompt leads to improved accuracy with regards to identifying the correct causing condition for said human while it is irrelevant whether the SDM has knowledge of the fact that the primary causing conditions are ordered by their respective primary causing condition probabilities. In other words, it is not critical if the prompt also includes the associated primary causing probabilities or not.

In general, prompt engineering refers to techniques for designing the prompt fed into an LLM such that it improves task performance. In principle, one could put the entirety of the PDM's condition space into the prompt, which would amount to using about 5000 additional tokens for a condition space of ^{~}1200 conditions (and potentially many more for larger ontologies such as the International Classification of Diseases, Version 10 (ICD10), made available via UMLS), and ask the LLM to adhere to the PDM's condition space. By limiting the candidate conditions put in the prompt to relevant causing conditions determined by the PDM improves diagnostic accuracy compared to a static condition list. Further, by only using a limited number of additional tokens, the herein disclosed approach leads to faster and cheaper inference compared to a static condition list.

In one embodiment, the secondary diagnosis step may use further LLMs as further SDMs. In this embodiment, each LLM is provided with at least a subset of the outputs of the remaining diagnosis models so that they can operate in parallel or sequential configurations. An example for a parallel configuration is that the medical state indicators and list of relevant primary causing conditions from the PDM are not only passed to a first SDM but concurrently also to at least a second SDM. An example for a sequential configuration is that a subset of the output from the first SDM (an ordered condition list of relevant secondary causing conditions, wherein relevance of a secondary causing condition is defined in analogy to the primary causing conditions) is passed together with the obtained medical state indicators to at least a second SDM. That is, in the sequential configuration, the information flows from the PDM to the first SDM to the second SDM, and so on, while in the parallel configuration the information is flowing from PDM to multiple SDMs.

In one embodiment, the SDM output is merged with the PDM output. For merging these outputs, the secondary causing condition probabilities are normalized in relation to the primary causing condition probabilities. It is to be noted that the probabilities determined by the PDM are typically normalized and may be distributed amongst more than 1000 conditions. On the other hand, the probabilities derived by the LLM are typically distributed amongst a much smaller number of conditions (typically in the order of five) and their relative values are typically more reliable than the absolute values. To make probabilities of the PDM comparable with the probabilities of the SDM, the respective LLM probabilities can be normalized such that their sum is equal to the sum of the PDM probabilities for a same number of top conditions (typically about five unless the LLM generates a shorter list).

After the normalization step, PDM and SDM probabilities are averaged for same conditions. In other words, average causing condition probabilities are computed by averaging the primary and secondary condition probabilities which relate to a same causing condition. A person skilled in the art may apply alternative normalization and averaging techniques.

As a final step for merging PDM and SDM outputs, a consolidated causing condition list with possible causing conditions for said human sorted in descending order of the associated averaged causing condition probabilities can be used to create an output to the human user. The consolidated causing condition list has an accuracy with regard to the underlying cause of the user's symptom(s) which is higher than the PDM and SDM outputs alone, respectively. In other words, the consolidated causing condition list provides more reliable diagnosis support characterizing the user's medical state with regard to possible medical causes for the user's symptoms. The diagnosis support information provided by the consolidated causing condition list can also support a medically trained person to select an appropriate therapeutic treatment for the human user.

In general, "classic" ensembling methods (such as "staking") are concerned with taking the individual results of different algorithms and combining them into a single result. This is possible since the algorithms of the ensemble are designed to use the same input and output space. For example, in case of condition suggestions, this may be an agreed condition ontology. However, if models are not operating on the same output space (like PDM and SDM), it is not possible to easily determine whether the individual models mean the same thing (e.g., PDM: "upper respiratory infect" vs. SDM: "common cold") and whether they are operating on the same level of granularity (e.g.: PDM: "Diabetes" vs. SDM: "Diabetes type II"). This typically results in a very complex mapping between the condition spaces. However, since the condition space of the LLM (SDM) is not well defined (i.e., it may not strictly adhere to existing ICD10 conditions and may forget existing conditions or invent new ones due to hallucinations), this problem is compounded in an ensemble involving at least one LLM. Usually in an ensemble, all results are computed independently, i.e. model A does not - or rather should not - influence the computation of model B. Practically, the computation can be parallelized.

In the herein disclosed "alternative" ensembling, the condition space problem of the merging step is solved by constraining the LLM output to the relevant primary causing conditions of the PDM. The PDM has a defined output space, whereas the LLM (SDM) does not have a defined output space. The PDM dynamically constrains the LLM output. For this purpose, an ordered list of the PDM is provided to the SDM. That is, the computation is not simultaneous but sequential, and the models are not independent of each other. As a consequence, the herein disclosed approach is not "classic" ensembling but rather "chaining/sequencing" followed by ensembling. Although the LLM output is practically not fully deterministic, the noise of the LLM's non-determinism is smoothened by merging the PDM and SDM results before showing the consolidated causing condition list to the user.

Since a consolidated causing condition list and associated averaged causing condition probabilities are provided as output of an embodiment of the herein disclosed method, and each individual consolidated causing condition has a certain urgency and severity associated with it (e.g., ranging from "Self-care" to "Call ambulance"), this embodiment can also be applied to triage and care navigation, i.e., navigating the human to the appropriate medical care at the right time based on the urgency and severity associated with the consolidated causing conditions and their associated averaged causing condition probabilities. For deriving care advice to users, several approaches are possible including considering the highest care navigation level as recommendation (e.g., when "See family doctor next week", "Self-care", "Call ambulance" are the set of associated care advice levels, the recommendation to the user would be "Call ambulance").

In one embodiment, the merging function may not only be applied to the PDM and SDM outputs at the end of a question-flow, but after some responses to individual questions of the question-flow (e.g., even after each question of the question-flow). In this embodiment, the selection of the next question may depend on the consolidated output associated with the medical state indicators collected by the question-flow up to this point.

In one embodiment, the consolidated causing condition list may be used by the PDM to add one or more additional questions to the question-flow for collecting further evidences.

In one embodiment, a computer program product is provided for supporting medical diagnosis of a human with symptoms associated to the human's medical condition. The program, when loaded into a memory of a computing device and executed by at least one processor of the computing device, executes the steps of the herein disclosed computer-implemented method.

In one embodiment, a computer system is provided for supporting medical diagnosis of a human with symptoms associated to the human's medical condition. The system has a primary diagnosis model adapted to determine, from a set of predefined medical conditions, one or more possible primary causing conditions with associated primary causing condition probabilities for said human. Thereby, medical state indicators comprising said symptoms obtained from said human are used as inputs of the primary diagnosis model. Thereby, a particular relevant primary causing condition is associated with a particular relevant primary causing condition probability if the associated primary causing condition probability is above a predefined threshold or belongs to a predefined number of highest ranking primary causing condition probabilities. The system further has at least one Large Language Model used as secondary diagnosis model. The LLM is prompted to perform the role of a medical practitioner. Thereby, it is adapted to receive the obtained medical state indicators and at least one or more relevant primary causing conditions as part of a prompt. The secondary diagnosis model is instructed to generate an output of one or more possible secondary causing conditions with associated secondary causing condition probabilities for said obtained medical state indicators in conformance with classes defined by the one or more relevant primary causing conditions in the prompt.

In one embodiment, the system may further include a merging module adapted to merge the output of the secondary diagnosis model with the output of the primary diagnosis model. The merging can be achieved by:
- normalizing the secondary causing condition probabilities in relation to the primary causing condition probabilities;
- computing average causing condition probabilities by averaging the primary and secondary condition probabilities which relate to a same causing condition; and
- outputting a consolidated causing condition list with possible causing conditions for said human sorted in descending order of the associated averaged causing condition probabilities. Optionally, the merging module may also provide the associated averaged causing condition probabilities to said human.

Further aspects of the invention will be realized and attained by means of the elements and combinations particularly depicted in the appended claims. It is to be understood that both, the foregoing general description and the following detailed description are exemplary and explanatory only and are not restrictive of the invention as described.

### Short description of the figures

FIG. 1 shows a block diagram of a computer system for supporting medical diagnosis of a human with symptoms associated with the human's medical condition according to an embodiment;
FIG. 2A is a simplified flowchart of a computer-implemented method for supporting said medical diagnosis of a human according to an embodiment;
FIG. 2B is a continuation of the simplified flowchart of the computer-implemented method with additional steps according to an embodiment;
FIGs. 3A to 3C illustrate examples of graphical user interfaces which may be used for capturing human user evidence via a question-flow;
FIG. 4 illustrates condition list output examples at various stages of the computer-implemented method;
FIG. 5 shows an example of a graphical user interface which may be used for capturing free-text information about a patient's symptoms;
FIG. 6 is a simplified flow chart of an alternative prompting approach for avoiding LLM hallucinations; and
FIG. 7 is a diagram that shows an example of a generic computer device and a generic mobile computer device, which may be used with the techniques described herein.

### Detailed description

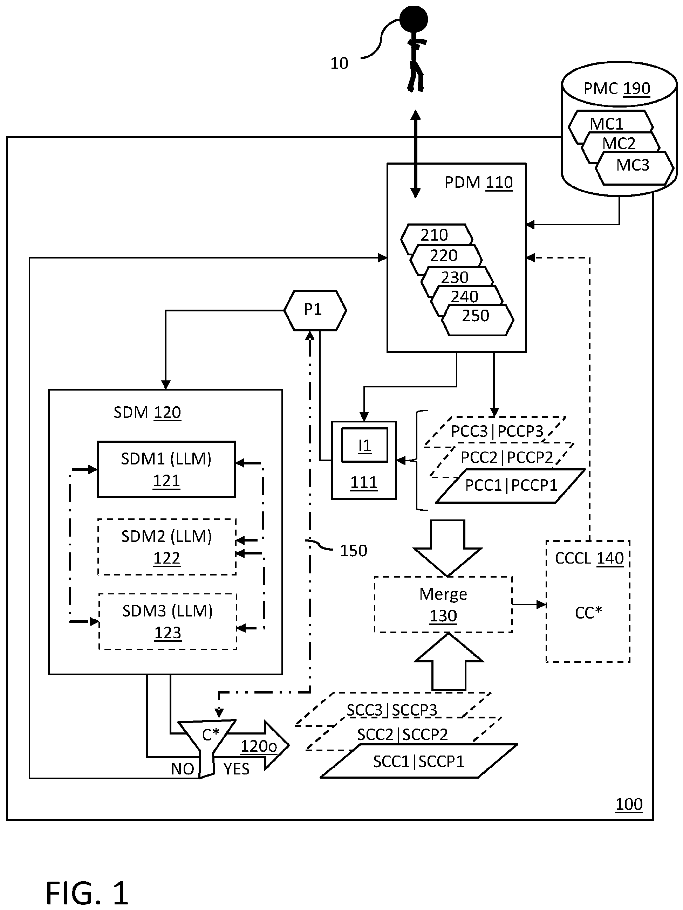

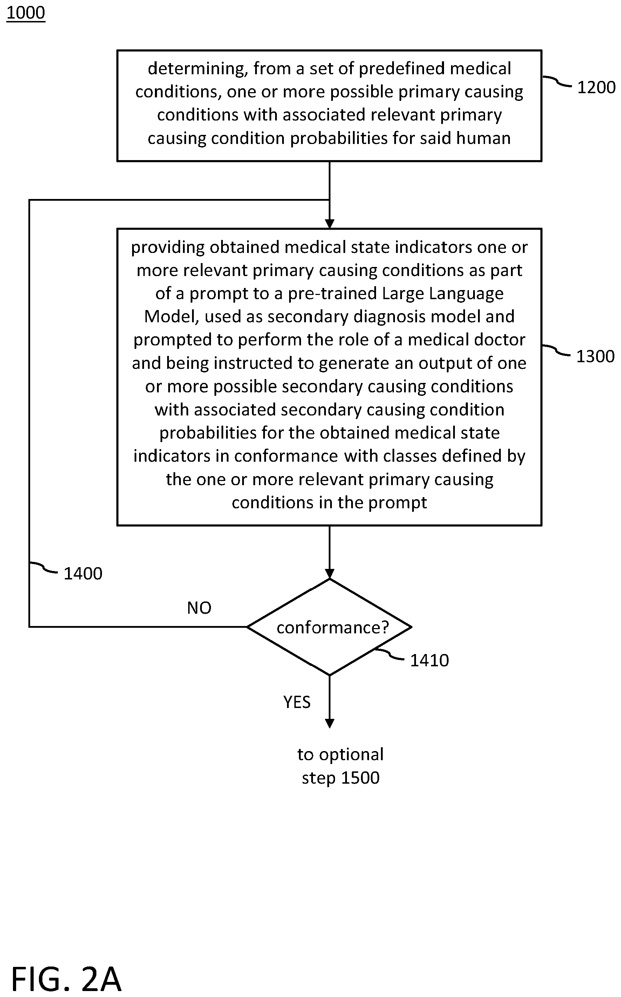

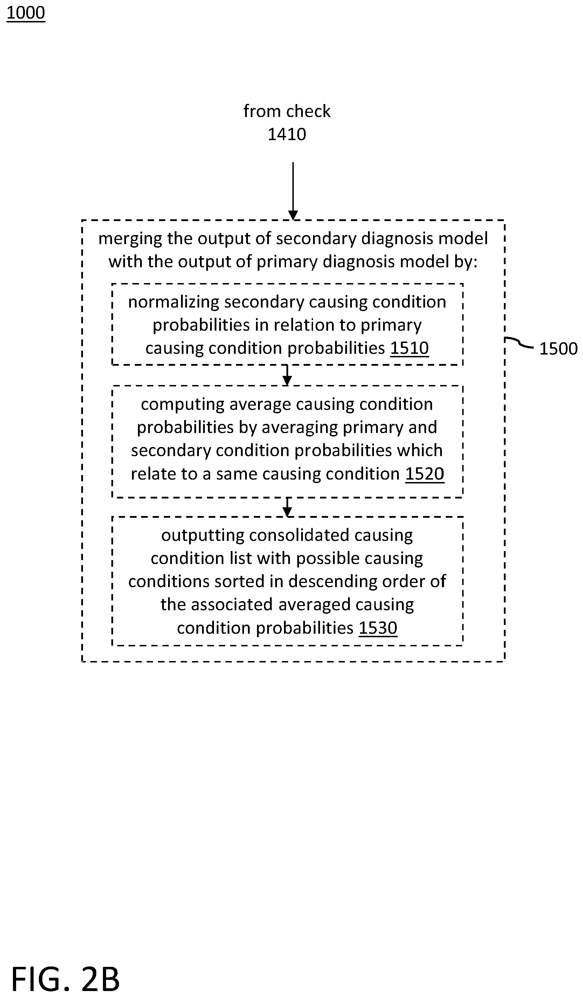

FIG. 1 shows a block diagram of an example embodiment of a computer system 100 for supporting medical diagnosis of a human 10 with symptoms associated to the human's medical condition. A user of system 100 may be the human patient 10 or a user taking care of said human. The user can interact with system 100 via a human-machine interface (illustrated by bold bidirectional arrow) which allows the user to provide data inputs to system 100 and receive system outputs (e.g., via a graphical user interface or audio interface). For example, inputs by the user and outputs to the user may be provided via a wearable computing device (e.g., a smartphone or tablet computer), which acts as a frontend of system 100. FIGs. 2A, 2B illustrate a simplified flowchart of a computer-implemented method 1000 for supporting said medical diagnosis of the human 10. The computer-implemented method can be executed by system 100. Therefore, the following description describes system 100 in view of the method 1000 and refers to reference numbers of all respective figures. Dashed lines in FIGs. 1, 2A, 2B represent optional system components/method steps used by optional embodiments.

System 100 has a primary diagnosis model PDM 110. PDM 110 is adapted to determine 1200, i.e., one or more possible primary causing conditions PCC1 to PCC3 with associated primary causing condition probabilities PCCP1 to PCCP3 for said human 10. The one or more possible primary causing conditions are thereby determined from a set PMC 190 of predefined medical conditions MC1, MC2, MC3. The predefined conditions may be stored by storage means being an integrated component of system 100, or they may be stored at a remote storage location accessible by system 100. Medical state indicators 210 to 250 comprising said symptoms obtained from said human are used as inputs of PDM 110. For example, the medical state indicators may be obtained in a question-flow where the human answers symptom related questions of PDM 110. PDM 110 may present a series of questions to the human 10 which are answered by the human typically by "yes" or "no" or "I don't know". For example, when using the above-mentioned Ada symptom checker application as PDM 110, after every answer by the human, the application calculates the next best question given the assessment of the current state to gather the most informative evidences. For example, one or more of the plurality of medical state indicators may be obtained by a probabilistic graphical model which uses information gain for choosing an appropriate next question in a questionnaire process applied to the human 10 to obtain the one or more medical state indicators 210 to 250. A person skilled in the art may use other implementations for PDM 110, such as for example, a decision tree, a support vector machine, a k-nearest neighbors classifier algorithm, or a logistic regression.

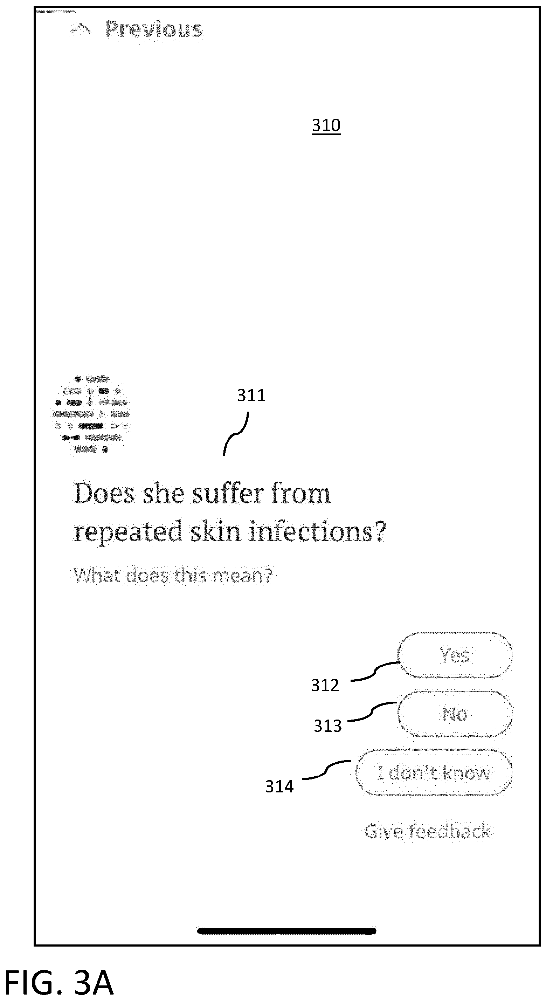

Once the question-flow is complete, a PGM (like the Ada symptom checker) computes a list of possible conditions rated by probability usually ranging from less than a dozen to a few hundred conditions) but typically only shows a subset of them (usually 3-5 conditions) to the user (e.g., via a GUI). The full list of possible conditions includes the relevant primary causing conditions for the human based on the determined evidences. Thereby, a particular possible primary causing condition PCC1 is considered relevant if it is associated with a relevant particular primary causing condition probability PCCP1. This is the case when the associated primary causing condition probability PCCP1 is above a predefined threshold or belongs to a predefined number of highest ranking primary causing condition probabilities. FIGs. 3A to 3C illustrate a part of an example question-flow with a respectively determined list of relevant particular primary causing conditions.

System 100 further has a prompt generator module 111 which is adapted to compose a prompt P1 for an LLM with at least the following prompt elements: an instruction I1 to the LLM to perform the role of a medical practitioner, the obtained medical state indicators 210 to 250, and at least one or more relevant primary causing conditions PCC*. The prompt P1 is then provided 1300 as input to at least one pre-trained LLM 121, 122, 123. Thereby, the LLM may be a standard LLM, such as GPT-4 from OpenAl. It may also be a model which has been trained more specifically with respective medical documents, such as MedLM from Google. The one or more LLMs are used as secondary diagnosis model 120. It is to be noted that a particular LLM 121 corresponds to a particular secondary diagnosis model SDM1 121. Thereby, the term LLM, as used herein, relates more to the technical implementation of the model, whereas SDM relates more to the medical purpose of said same model.

SDM1 is pre-trained such that it can slip into the role of a medical practitioner in response to the corresponding prompt element. Further, the prompt instructs the LLM(s) to use the obtained medical state indicators 210 to 250 and the at least one or more relevant primary causing conditions PCC* of the prompt P1 to generate an output 120o of one or more possible secondary causing conditions SCC1 to SCC3 with associated secondary causing condition probabilities SCCP1 to SCCP3 for said obtained medical state indicators 210 to 250. The one or more relevant primary causing conditions in the prompt P1 are used 150 as a guardrail function for the LLM(s) to ensure that the LLM output(s) is/are in conformance with classes C* defined by said relevant primary causing conditions.

Advantageously, the prompt P1 provides the relevant primary causing conditions to the SDM in descending order (e.g., PCC1 → PCC2 → PCC3 → ...) of the associated relevant primary causing condition probabilities (e.g., PCCP1 → PCCP2 → PCCP3 → ...). Surprisingly, it has been shown that the accuracy of the generated output 120o with one or more possible secondary causing conditions is improved with such an ordered list of relevant primary causing conditions being part of the prompt even without providing the associated probabilities in the prompt or stating in the prompt that the relevant primary causing conditions are ordered.

A checker function 150 (dashed double-dotted bidirectional arrow) of system 100 checks 1410 if the generated LLM output 120o is indeed in conformance with the classes C*. If it fails to limit the one or more possible secondary causing conditions to the classes C* defined by the one or more relevant primary causing conditions in the prompt P1, the checker function 150 triggers reapplying 1400 the prompt P1 to the LLM(s) until the generated output is in conformance (YES) with the classes C*. This checker function ensures that hallucinations are avoided which is mandatory in the field of medical applications to minimize the risk for humans that a wrong treatment option would be selected based on a wrong hallucinated secondary causing condition which could eventually worsen the state of health of the human. An abort criterion (e.g., a maximum number of retries) can be defined so that in case the LLM eventually fails to provide an output in conformance with the constraints set by the classes C*, the request is considered as failed and the system falls back to the PDM by itself. In other words, if the system does not reach said conformance (NO) before the abort criterion is met, the PDM is triggered to generate a new set of relevant conditions (e.g., by obtaining additional evidences).

In one embodiment, the system 100 may use a plurality 120 of secondary diagnosis models comprising multiple LLMs serving as SDM1 121, SDM2 122 and SDM3 123. These LLMs can be used in a parallel configuration or in a sequential configuration. In the parallel configuration, each SDM* 121, 122, 123 is provided the output of the primary diagnosis PDM 110 (in parallel). In the parallel configuration, each SDM* provides its own output. For example, SDM 120 may compute an average over the PDM and SDM* outputs as the output 120o if the conformance is reached. A skilled person can select an appropriate averaging method. For example, for each relevant particular primary causing condition provided by the PDM the respective probability is taken. If the corresponding PDM class is part of any of the SDM outputs, the associated secondary causing condition probability is added to the corresponding primary causing condition probability. In case the class occurs in multiple SDM outputs, the respective probability for each of the SDMs where it occurs is added. If said PDM class does not occur in an SDM output, a default probability of '0' is added. The cumulated probability values are then divided by the number of models (e.g., 1 PDM + 3 SDMs = 4) resulting in averaged probability values for each class.

In the sequential configuration, each SDM* 121, 122, 123 is provided with at least a subset of the outputs of the remaining secondary diagnosis models, as illustrated by the dash-dotted bidirectional arrows between the SDMs. For example, a first SDM (e.g., SDM1) receives the prompt P1 from the PDM and can provide its output as an input to the further SDMs 122, 123. SDM2 may again provide its output to SDM3. In this sequential configuration, only the last SDM in the sequence may provide the output 120o.

Using LLM(s) as SDM(s) has the further advantage that the obtained medical state indicators 210 to 250 may also include other modalities supported by the SDM such as free-text data, image data, audio data, or video data. An LLM can use such data for generating its secondary causing conditions and associated secondary causing condition probabilities.

In one embodiment, the system 100 may further include a merging module 130 which can merge 1500 the output of SDM 120 with the output of PDM 110. This can be achieved by: normalizing 1510 the secondary causing condition probabilities in relation to the primary causing condition probabilities; computing 1520 average causing condition probabilities by averaging the primary and secondary condition probabilities which relate to a same causing condition; and outputting 1530 a consolidated causing condition list CCCL 140 with possible causing conditions CC* for said human 10 sorted in descending order of the associated averaged causing condition probabilities. Optionally, the system may also output the associated averaged causing condition probabilities.

In the following, two example cases are described in detail. In the first example, the impact on the ranking of a particular condition (PFAPA syndrome) is demonstrated for each stage of the herein disclosed method. In the PDM assessment, PFAPA syndrome, which is actually the correct condition in the example, is on rank 3. In the SDM assessment it is on rank 2 and after the optional merging step the ensemble brings it up to the top position on rank 1. In contrast, when using an LLM which is not constrained by the PDM output, the correct condition did not show up amongst the top 5 suggested conditions.

FIG. 3A illustrates an example of a graphical user interface (GUI) 310 which is used to implement the question-flow used by the PDM. A question 311 is shown to the user to obtain information regarding repeated skin infections of the patient. The user can simply answer such questions by pushing 'Yes' 312 or 'No' 313 buttons. In case of being unsure, the user can also push the 'I don't know' button 314. Once the PDM has collected all necessary information via the question-flow, the prompt generator composes the prompt to be provided as input to the LLM (SDM). The following example prompt starts with instructing the LLM that it plays the role of a medical assistant and the task to be performed. Then, the list of potential conditions determined by the PDM is provided as the guardrail function. Further, the output format of the LLM is defined. Finally, the collected patient information regarding the patient's evidences is included.

### Example prompt EP1 (in italics):

*You are a medical assistant. Given the clinical vignette of a patient, provide a list with up to 5 potential medical causes, sorted by decreasing likelihood.*

*Provide the likelihood of each condition in %. Note that the sum of the likelihoods in the list must not be higher than 100%.*

*For each condition indicate its name, which must be among the following list:*
- *Chronic granulomatous disease*
- *Non-Hodgkin's lymphoma*
- *PFAPA syndrome*
- *Hodgkin's lymphoma*
- *Hyperimmunoglobulinemia D with periodic fever*
- *Chronic fatigue syndrome*
- *Cytomegalovirus infection*
- *TNF receptor associated periodic syndrome*
- *Familial mediterranean fever*
- *Pediatric HIV infection*
   ...

*It is important that you provide all the required information in the following JSON format*

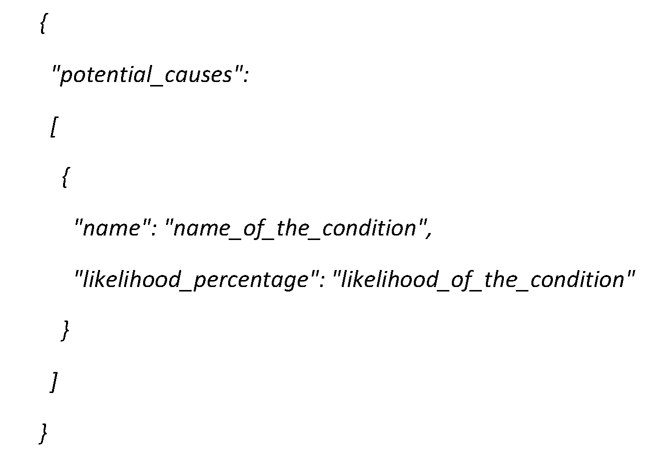

### ### Clinical vignette

*The patient is a 4 years old female.*

### Previous medical history:

*diabetes: no*
*recent exposure to ticks: no*
*Mediterranean descent: no*

*The patient's primary complaint is: recurring episodes of feverish illness*
*pain in multiple joints*
*further specifying pain in multiple joints, time since onset: one month to one year*
*further specifying pain in multiple joints, physical exertion: no effect*

*The patient's current symptoms are:*
*recurrent skin infections: no*
*repeated respiratory infections: no*
*fatigue: no*
*fever: yes*
*knee pain: no*
*pale face: no*
*morning stiffness: no*
*lump under the skin on the neck: yes*
*further specifying lump under the skin on the neck, time since onset: one month to one year*
*further specifying lump under the skin on the neck, tenderness: yes*
*further specifying lump under the skin on the neck, change in size: no*
*prolonged nighttime sleep: no*
*reddened throat: no*
*diarrhea: no*
*chills: no*
*generalized muscle pain: no*
*widespread redness of the skin: no*
*sores inside the mouth: yes*
*sore throat: no*
*vomiting: no*
*painful mouth: no*

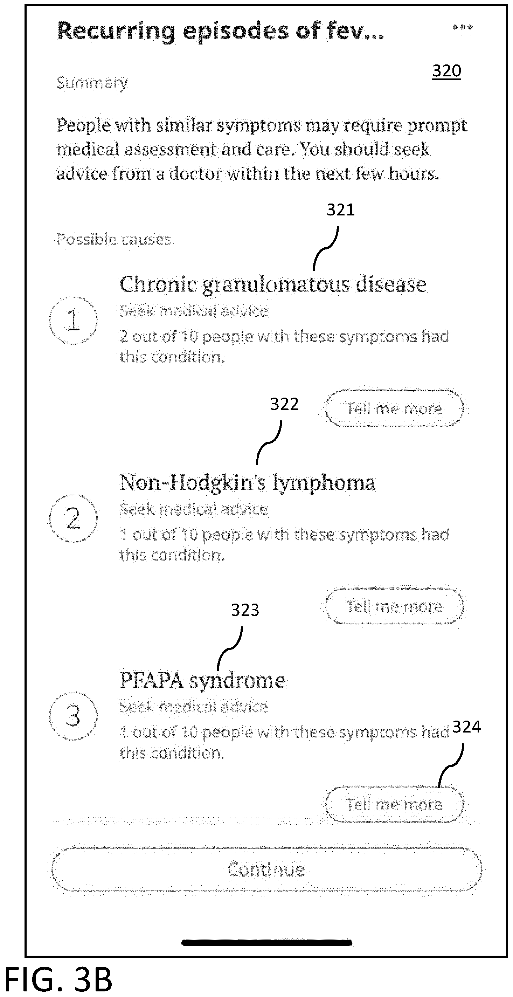

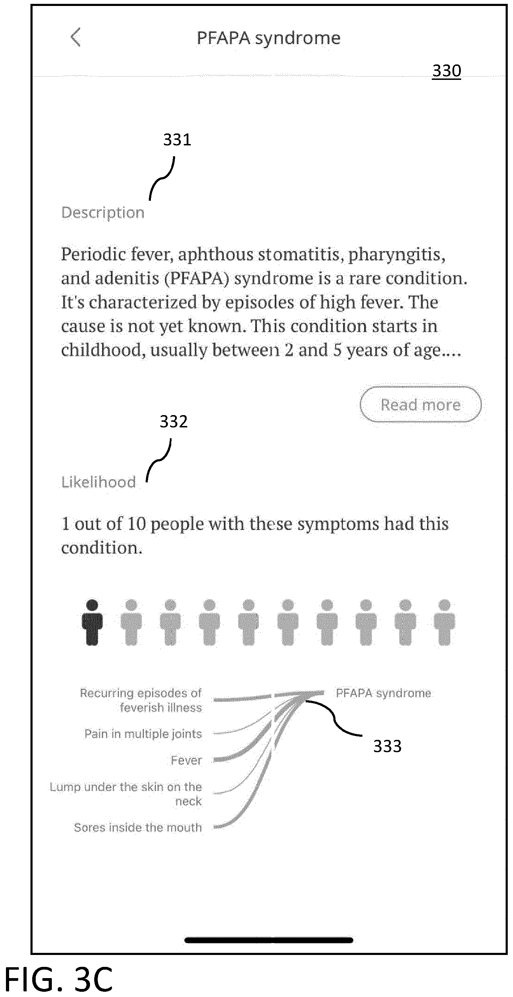

It is to be noted, that in the real example, the PDM provided a list of the top 100 predefined medical conditions ranked by their respective probabilities. The example prompt only shows the top 10 of this top 100 list. FIG. 3B is a GUI example 320 of how the PDM output may be presented to the user. The three top ranking conditions 321, 322, 323 are shown to the user with some comments and recommendations. When clicking on the 'Tell me more' button 324, the user is provided with detailed information 330 about the respective condition as shown in FIG. 3C. A short description 331 is given together with the information regarding the likelihood 332 and how the main symptoms contribute to the primary causing condition candidate 333.

The SDM answer of the LLM to the above prompt is:

```
 {
 "potential_causes":
 [
  {
   "name": "Systemic-onset juvenile idiopathic arthritis",
   "likelihood_percentage": "30"
  },
  {
   "name": "PFAPA syndrome",
   "likelihood_percentage": "25"
  },
  {
   "name": "Non-Hodgkin's lymphoma",
   "likelihood_percentage": "20"
  },
  {
   "name": "Hodgkin's lymphoma",
   "likelihood_percentage": "15"
  },
  {
   "name": "Juvenile idiopathic arthritis",
   "likelihood_percentage": "10"
  }
 ]
 }
```

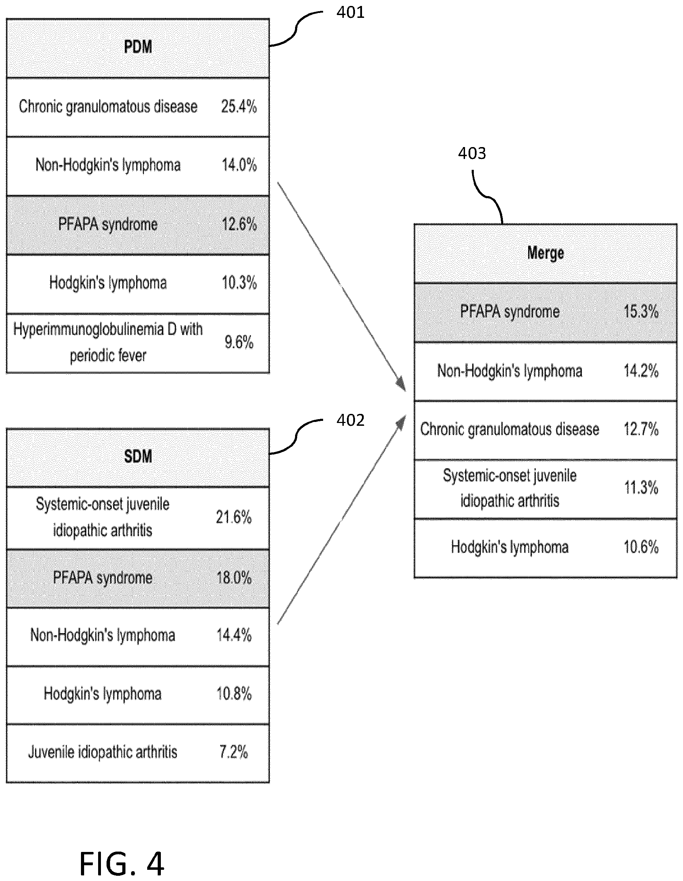

FIG. 4 illustrates the ranking of the true condition *('PFAPA syndrome')* in the respective condition ranking list together with the respective condition probabilities after the various processing stages. The condition list 401 after PDM processing includes *'PFAPA syndrome'* on rank 3 with a probability of 12.6 %. The condition list 402 after SDM processing and applying 1510 includes *'PFAPA syndrome'* on rank 2 with a probability of 18.0 %. The consolidated condition list 403 after merging step includes *'PFAPA syndrome'* on rank 1 with a probability of 15.3 %. It is to be noted that the condition lists after PDM and SDM processing (and also after the optional merging step) do not only differ in the order of the top five conditions but also differ with regard to the content of the top five conditions.

Sometimes, the LLM may be prompted with medical terms which are not known by the LLM in its current training state. In such cases it can be advantageous to further specify the generated prompt by adding more context to the conditions provided in the PDM condition list. In other words, a definition may be added to the respective generated prompt EP1. The following example prompt EP1a shows how the initial elements of EP1 can be substituted by elements providing additional condition context:

### Example prompt EP1a (in italics):

*You are a medical assistant. Given the clinical vignette of a patient, provide a list with up to 5 potential medical causes, sorted by decreasing likelihood.*

*Provide the likelihood of each condition in %. Note that the sum of the likelihoods in the list must not be higher than 100%.*

*For each condition indicate its name, which must be among the following list* of *allowed condition names:*

*The allowed list is structured as pairs ("name_of_the_condition": "description_of_the_ condition "):*
- *"Chronic granulomatous disease": chronic granulomatous disease (cgd) is a rare, primary immunodeficiency disorder characterized by a congenital deficiency of phagocyte nicotinamide adenine dinucleotide (nadph) oxidase activity, resulting in impaired neutrophil function and recurrent infections with catalase-positive organisms.*
- *"Non-Hodgkin's lymphoma": non-hodgkin's lymphoma (nhl) is a heterogeneous group of malignant neoplasms arising from abnormal proliferation of mature b-cell or t-cell lymphoid lineage, characterized by the presence of reed-sternberg-like cells and*/*or atypical lymphoid infiltrates in various anatomic sites, including lymph nodes, spleen, liver, and extranodal locations.*
- *"PFAPA syndrome": pfapa syndrome is a rare condition characterized by recurring episodes of high fever, aphthous stomatitis, pharyngitis, and adenitis, with symptoms including mouth ulcers, sore throat, and swollen lymph nodes, with most people returning to good health between episodes.*
- *"Hodgkin's lymphoma": hodgkin's lymphoma is a type of malignant neoplastic disease characterized by the proliferation of abnormal reed-sternberg cells and nodular sclerosis, typically affecting the lymph nodes and sometimes involving other lymphoid tissues or organs.*
- *"Hyperimmunoglobulinemia D with periodic fever": hyperimmunoglobulinemia d with periodic fever (hids) is a rare genetic disorder characterized by recurrent episodes of high fever, joint pain, and lymphadenopathy, typically starting in early childhood.*
- *"Chronic fatigue syndrome": chronic fatigue syndrome is a complex, multifaceted disorder characterized by persistent and relapsing symptoms of profound fatigue, often accompanied by orthostatic intolerance, cognitive impairment, and musculoskeletal pain, which persists for at least six months without identifiable underlying cause or explanatory medical condition.*
- *"Cytomegalovirus infection": cytomegalovirus infection is a ubiquitous, self-limiting viral illness primarily affecting the respiratory tract, characterized by flu-like symptoms, that can reactivate in individuals with compromised immune function, including those with immunosuppressive therapy or underlying medical conditions. let me know if you'd like me to clarify anything!*
- *"TNF receptor associated periodic syndrome": tnf receptor associated periodic syndrome' is a rare, autosomal dominant genetic disorder characterized by recurrent episodes of inflammation driven by mutations in the tnfrsf1a gene, presenting as fever, rash, musculoskeletal pain, and other systemic symptoms.*
- *"Familial mediterranean fever": familial mediterranean fever is a rare, autosomal recessivemediated, recurrent inflammatory disorder characterized by episodic fever and multi-organ involvement, primarily affecting individuals of mediterranean origin.*
- *"Pediatric HIV infection": refers to a human immunodeficiency virus (hiv) infection acquired during childhood, often through vertical transmission from an infected mother or breastfeeding. the clinical presentation is characterized by rapid disease progression due to the immature immune system in children, with symptoms such as recurrent infections, diarrhea, and poor weight gain or growth.*
- *"Ulceroglandular tularemia": an infection caused by francisella tularensis bacteria, characterized by the development of an open sore (ulcer) at the site of inoculation, accompanied by swollen lymph nodes, fever, headaches, chills, and exhaustion.*
   ...

A second example shows how additional free-text input processed by the LLM (in the example: GPT-4 from OpenAl) can improve the overall list of suggested conditions for the ensemble.

This specific second example, presents a medically unspecific case: a child refusing to eat for a short period of time, having no other complaints. The diagnosis possibilities are wide, including a non-pathological cause. Situational or circumstantial evidence from personal history is hard to model into medical knowledge but is usually part of a patientphysician conversation and is in this case useful if provided in free-text format to the LLM. The free-text for this example is: *"I'm afraid she might have swallowed a toy."*

In this example, the (simplified) example prompt EP2 includes:
*You are a medical assistant. Given the clinical vignette of a patient, provide a list with up to 5 potential medical causes, sorted by decreasing likelihood.*

*Provide the likelihood of each condition in %. Note that the sum of the likelihoods in the list must not be higher than 100%.*

*For each condition indicate its name, which must be among the following list:*
- *Viral stomach bug in children*
- *Teething*
- *Dental cavity*
- *Roseola infantum*
- *Iron deficiency anemia*
   ...
- *Foreign body ingestion*
   ...

*It is important that you provide all the required information in the following JSON format*

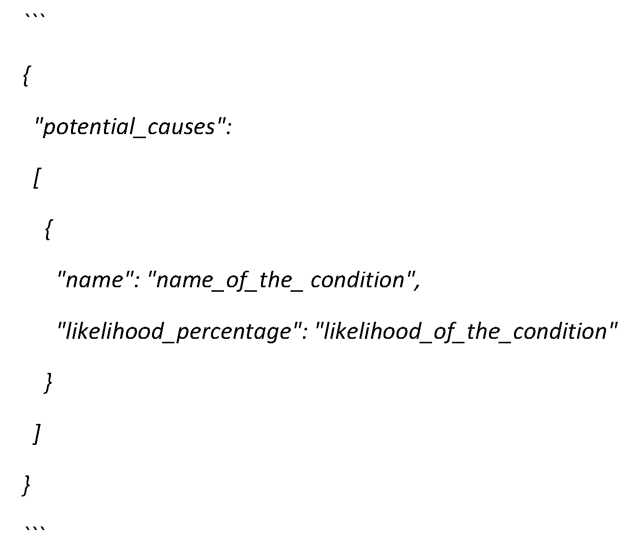

### ### Clinical vignette

*The patient is a 2 years old female.*

### Previous medical history:

*skin injury: no*
*head or neck injury: no*

*The patient's primary complaint is: feeding difficulties*
*further specifying feeding difficulties, time since onset: less than one day*

*The patient's current symptoms are:*
*diarrhea: no*
*patches inside the mouth: no*
*rapid pulse: no*
*abdominal pain: no*
*nostrils flaring: no*
*sores inside the mouth: no*
*fever: no*
*teething: no*
*cough: no*
*nausea: no*
*difficulty breathing: no*
*runny nose: no*
*rash on the trunk: no*
*fatigue: no*
*bruise on the scalp: no*
*painful urination: no*
*less energetic or less alert infant: no*
*earache: no*

### ### Additional information from patient

*"I'm afraid she might have swallowed a toy."*

In this second example, the top five in the condition list provided by the PDM are shown in table 1. It is to be noted that the rank of the true condition was '58'. In the example, the top 100were provided to the LLM in the respective ranking order (but without providing the condition probabilities).

**Table 1 - PDM condition list (top five)**

| **condition candidate** | **condition probability** |
|---|---|
| Viral stomach bug in children | 13.6% |
| Teething | 9.9% |
| Dental cavity | 7.9% |
| Roseola infantum | 6.7% |
| Tron deficiency anemia | 6.5% |

The SDM answer of the LLM to the above prompt including the free-text portion (and after normalization) is shown in table 2:

**Table 2 - SDM condition list**

| **condition candidate** | **condition probability** |
|---|---|
| Foreign body ingestion | 26.7% |
| Pediatric gastroesophageal reflux disease | 8.9% |
| Constipation | 4.5% |
| Non-specific symptoms | 2.2% |
| Failure to thrive, not further specified | 2.2% |

The true condition *'Foreign body ingestion'* already shows up on rank '1'. Also, after the merging step, the true condition remains on rank '1' as shown in table 3:

**Table 3 - merged condition list**

| **condition candidate** | **condition probability** |
|---|---|
| Foreign body ingestion | 13.4% |
| Viral stomach bug in children | 6.8% |
| Pediatric gastroesophageal reflux disease | 5.7% |
| Teething | 4.9% |
| Dental cavity | 4.0% |

When prompting the LLM with the condition list provided by the PDM but without including the free-text element in the above prompt example, the LLM condition list does not include the true condition as shown in table 5:

**Table 5 - SDM condition list without free-text prompt element**

| **condition candidate** | **condition probability** |
|---|---|
| Non-specific symptoms | 13.4% |
| Pediatric gastroesophageal reflux disease | 11.1% |
| Constipation | 8.9% |
| Failure to thrive, not further specified | 6.7% |
| Iron deficiency anemia | 4.5% |

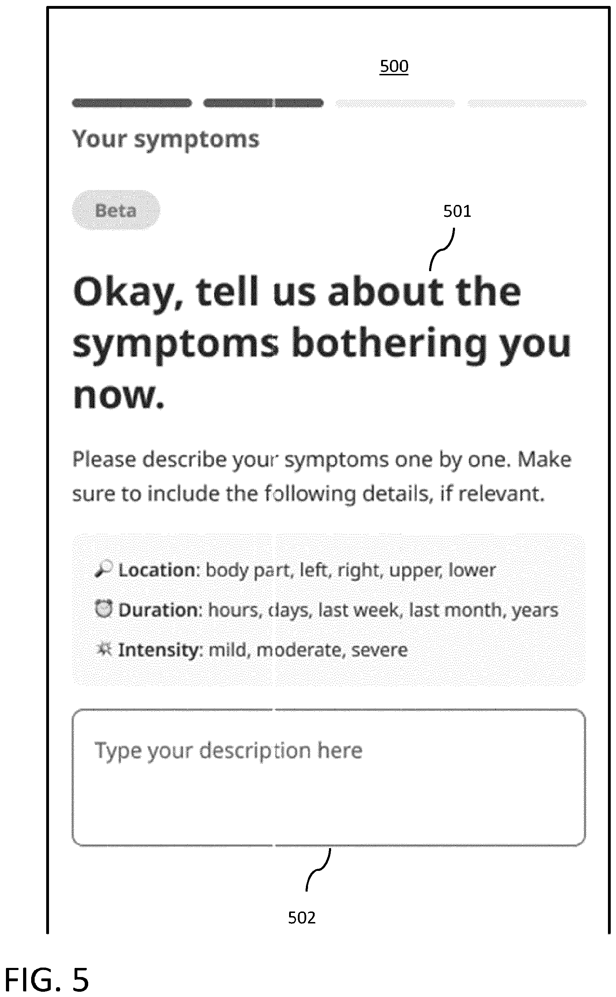

Even after a merging step of the LLM condition list of table 5 with the entire top 100 condition list provided by the PDM the rank of the true condition in the consolidated condition list remained on rank 58. That is, free-text elements in the prompt can substantially improve the accuracy of the ranking of conditions once processed by the SDM because LLMs are capable to deal with unstructured free-text inputs in a better way than the PDM which depends on structure input describing the human's evidences. FIG. 5 illustrates an example of a GUI 500 which can be integrated into the question-flow of the PDM to capture free-text elements for the prompt by asking a general question 501 and allowing the user to enter a free-text response, e.g., in description field 502.

Experimental evidence has been found that the herein disclosed approach with the described sequential processing using a PDM and subsequent SDM(s) results in a higher accuracy regarding the ranking of conditions in the resulting condition list such that the true conditions were found in higher ranked positions compared to using a PDM or SDM as a standalone ranking tool. This ranking accuracy can even be more improved when applying the merging step at the end. A further advantage of the disclosed approach is that the implementing computer system is frugal with compute resources and flexible.

To this end, quality metrics were calculated on a few hundred case vignettes, i.e. plausible patient encounters created by doctors (based on their clinical experience). These case vignettes have not been used for creating the PDM, i.e., there is no overfitting of these cases to the PDM symptom checker (and neither for the LLM since both are not in the public domain, i.e., they cannot be in the training set of LLMs like GPT-4). Note that the goldstandard true condition label is provided by the creator of the case.

The most important metric to be considered is M3. It describes whether in the condition list suggested by the claimed algorithm the true condition is among the first three conditions. Similarly, there are also other Mx metrics such as M1 (it is the first condition), M5 (it is among the top 5 conditions), etc. All described Mx metrics are ranging from 0% to 100% as the percentage of cases for which the true condition is among the top N suggestions. A more comprehensive metric is the "Discounted Cumulative Gain" (DCG), which basically takes into account all positions (M1-5) on which the correct condition might be suggested. The DCG metric ranges between 0 and 1, the higher the better.

Based on an evaluation with 605 vignettes and GPT-4, it can be shown that the claimed method and system:
- perform better than a simple mapping operation for the LLM outputs
- save computational cost and latency
- improve the practical non-determinism of a LLM stand-alone solution via the ensembling operation, i.e., the merging of PDM and SDM outputs.

Table 6 shows an overview for four different metrics and the accuracy achieved by the various methods for determining the conditions list for the patient.

**Table 6 - performance metrics overview**

| **metrics** | **PDM (only)** | **LLM (only)** | **PDM+LLM (top 100 candidates)** | **PDM+LLM (all candidates)** | **ensembled (top 100 candidates)** | **ensembled (all candidates)** |
|---|---|---|---|---|---|---|
| M1 | 38.0% | 37.7% | 40.5% | 40.3% | 40.0% | 40.2% |
| M3 | 55.4% | 55.7% | 58.7% | 61.3% | 61.0% | 62.6% |
| M5 | 63.0% | 62.5% | 65.1% | 66.6% | 66.1% | 67.3% |
| DCG | 0.515 | 0.509 | 0.537 | 0.548 | 0.544 | 0.553 |

It was shown that the combined PDM+LLM approach provides better accuracy than the PDM or LLM stand-alone solutions when including only the top 100 candidates (i.e., relevant primary causing conditions) of the PDM condition list in the LLM prompt and also when including all PDM candidates (i.e., non-zero posterior candidates) in the prompt. The accuracy is even further improved when applying the merging step in the ensembling embodiment.

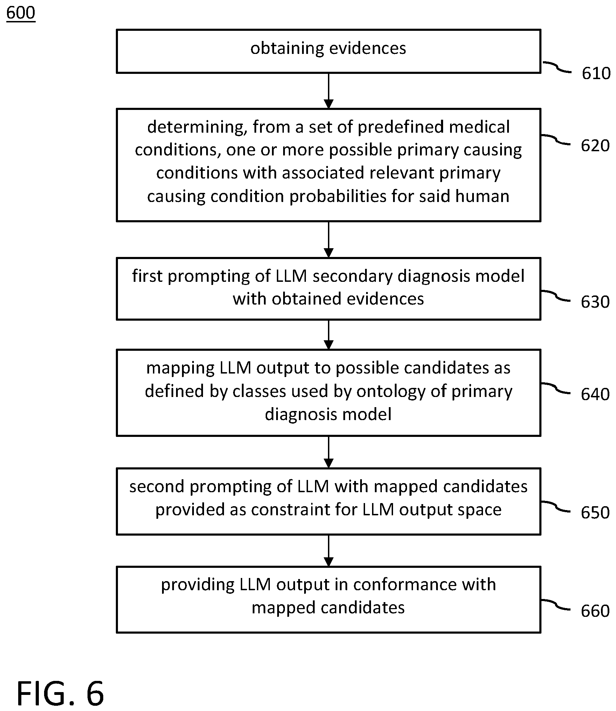

The herein disclosed concept can be slightly modified with regard to the generation of the prompt and the way how compliance of the LLM output(s) with meaningful classes is enforced to avoid hallucinations as illustrated in simplified flow chart 600 of FIG. 6. For example, the same LLM may be prompted twice. Thereby, the first prompt 630 includes the evidences obtained 610 for the human patient via the PDM. However, the first prompt does not restrict the LLM output space by the condition candidates determined 620 by the PDM. In other words, the PDM condition list output is not added to the first prompt but only the evidences. The LLM condition list output in response to the first prompt is then mapped 640 to possible candidates as defined by the entire condition classes which are used by the PDM's ontology. For example, such a mapping may be created via a k-nearest neighbors (k-NN) classifier that calculates the similarity between (word) embeddings of the PDM classes and the suggested conditions of the LLM. The similarity threshold (such as cosine similarity or Euclidean distance) is advantageously chosen in a lenient way, i.e., it is optimized for recall and not precision (i.e., in addition to the mapping between the correct condition class from the PDM ontology and the LLM candidate suggestion, the k-NN will map possibly one or more "false positive" PDM classes to each LLM candidate suggestion (low precision), however, the chances of missing the correct class from the PDM ontology amongst mapped LLM candidate suggestions is low (high recall)). Then, the same LLM is provided 650 with a second prompt where now the PDM-mapped candidates are provided as a constraint for the LLM output space. In other words, instead of directly using the PDM condition list output as constraint, the system firstly determines an unconstrained LLM output which is then mapped to the condition classes of the PDM ontology via an appropriate mapping mechanism. Finally, the mapping result (a subset of the PDM output space) is then used for defining the classes to which the LLM output in response to the second prompt has to adhere. That is, the condition suggestions provided 660 by the LLM in response to the second prompt are in conformance with the mapped candidates determined after the first prompt. In this modification, the dependency of the LLM on the PDM is reduced, i.e., both algorithms can run in parallel since the condition suggestions from the PDM output are not needed for prompting the LLM. Moreover, the LLM can also suggest conditions which were not considered by the PDM (e.g., due to inherent modelling short comings).

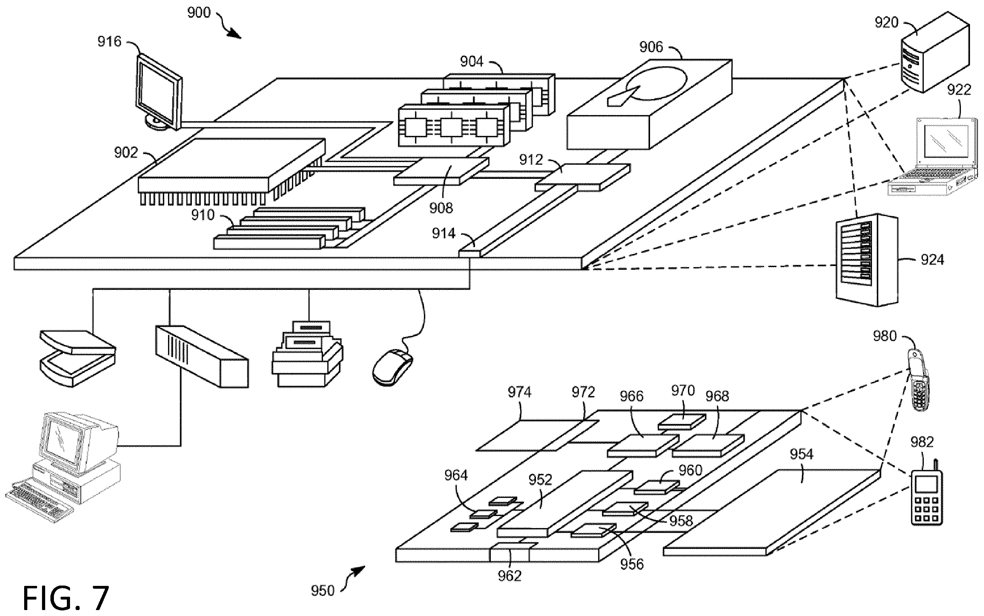

FIG. 7 is a diagram that shows an example of a generic computer device 900 and a generic mobile computer device 950, which may be used with the techniques described here. Computing device 900 is intended to represent various forms of digital computers, such as laptops, desktops, workstations, personal digital assistants, servers, blade servers, mainframes, and other appropriate computers. Generic computer device 900 may correspond to the computer system 100 of FIG. 1. Computing device 950 is intended to represent various forms of mobile devices, such as personal digital assistants, cellular telephones, smart phones, and other similar computing devices. For example, computing device 950 may be used as a frontend by a user to interact with the computing device 900. For example, device 950 may also provide the frontend to interact with the application. The components shown here, their connections and relationships, and their functions, are meant to be exemplary only, and are not meant to limit implementations of the inventions described and/or claimed in this document.

Computing device 900 includes a processor 902, memory 904, a storage device 906, a high-speed interface 908 connecting to memory 904 and high-speed expansion ports 910, and a low-speed interface 912 connecting to low-speed bus 914 and storage device 906. Each of the components 902, 904, 906, 908, 910, and 912, are interconnected using various busses, and may be mounted on a common motherboard or in other manners as appropriate. The processor 902 can process instructions for execution within the computing device 900, including instructions stored in the memory 904 or on the storage device 906 to display graphical information for a GUI on an external input/output device, such as display 916 coupled to high-speed interface 908. In other implementations, multiple processors and/or multiple buses may be used, as appropriate, along with multiple memories and types of memory. Also, multiple computing devices 900 may be connected, with each device providing portions of the necessary operations (e.g., as a server bank, a group of blade servers, or a multi-processor system).

The memory 904 stores information within the computing device 900. In one implementation, the memory 904 is a volatile memory unit or units. In another implementation, the memory 904 is a non-volatile memory unit or units. The memory 904 may also be another form of computer-readable medium, such as a magnetic or optical disk.

The storage device 906 is capable of providing mass storage for the computing device 900. In one implementation, the storage device 906 may be or contain a computer-readable medium, such as a floppy disk device, a hard disk device, an optical disk device, or a tape device, a flash memory or other similar solid-state memory device, or an array of devices, including devices in a storage area network or other configurations. A computer program product can be tangibly embodied in an information carrier. The computer program product may also contain instructions that, when executed, perform one or more methods, such as those described above. The information carrier is a computer- or machine-readable medium, such as the memory 904, the storage device 906, or memory on processor 902.

The high-speed controller 908 manages bandwidth-intensive operations for the computing device 900, while the low-speed controller 912 manages lower bandwidth-intensive operations. Such allocation of functions is exemplary only. In one implementation, the high-speed controller 908 is coupled to memory 904, display 916 (e.g., through a graphics processor or accelerator), and to high-speed expansion ports 910, which may accept various expansion cards (not shown). In the implementation, low-speed controller 912 is coupled to storage device 906 and low-speed expansion port 914. The low-speed expansion port, which may include various communication ports (e.g., USB, Bluetooth, Ethernet, wireless Ethernet) may be coupled to one or more input/output devices, such as a keyboard, a pointing device, a scanner, or a networking device such as a switch or router, e.g., through a network adapter.

The computing device 900 may be implemented in a number of different forms, as shown in the figure. For example, it may be implemented as a standard server 920, or multiple times in a group of such servers. It may also be implemented as part of a rack server system 924. In addition, it may be implemented in a personal computer such as a laptop computer 922. Alternatively, components from computing device 900 may be combined with other components in a mobile device (not shown), such as device 950. Each of such devices may contain one or more of computing device 900, 950, and an entire system may be made up of multiple computing devices 900, 950 communicating with each other.

Computing device 950 includes a processor 952, memory 964, an input/output device such as a display 954, a communication interface 966, and a transceiver 968, among other components. The device 950 may also be provided with a storage device, such as a microdrive or other device, to provide additional storage. Each of the components 950, 952, 964, 954, 966, and 968, are interconnected using various buses, and several of the components may be mounted on a common motherboard or in other manners as appropriate.

The processor 952 can execute instructions within the computing device 950, including instructions stored in the memory 964. The processor may be implemented as a chipset of chips that include separate and multiple analog and digital processors. The processor may provide, for example, for coordination of the other components of the device 950, such as control of user interfaces, applications run by device 950, and wireless communication by device 950.

Processor 952 may communicate with a user through control interface 958 and display interface 956 coupled to a display 954. The display 954 may be, for example, a TFT LCD (Thin-Film-Transistor Liquid Crystal Display) or an OLED (Organic Light Emitting Diode) display, or other appropriate display technology. The display interface 956 may comprise appropriate circuitry for driving the display 954 to present graphical and other information to a user. The control interface 958 may receive commands from a user and convert them for submission to the processor 952. In addition, an external interface 962 may be provide in communication with processor 952, so as to enable near area communication of device 950 with other devices. External interface 962 may provide, for example, for wired communication in some implementations, or for wireless communication in other implementations, and multiple interfaces may also be used.

The memory 964 stores information within the computing device 950. The memory 964 can be implemented as one or more of a computer-readable medium or media, a volatile memory unit or units, or a non-volatile memory unit or units. Expansion memory 984 may also be provided and connected to device 950 through expansion interface 982, which may include, for example, a SIMM (Single In Line Memory Module) card interface. Such expansion memory 984 may provide extra storage space for device 950, or may also store applications or other information for device 950. Specifically, expansion memory 984 may include instructions to carry out or supplement the processes described above, and may include secure information also. Thus, for example, expansion memory 984 may act as a security module for device 950, and may be programmed with instructions that permit secure use of device 950. In addition, secure applications may be provided via the SIMM cards, along with additional information, such as placing the identifying information on the SIMM card in a non-hackable manner.

The memory may include, for example, flash memory and/or NVRAM memory, as discussed below. In one implementation, a computer program product is tangibly embodied in an information carrier. The computer program product contains instructions that, when executed, perform one or more methods, such as those described above. The information carrier is a computer- or machine-readable medium, such as the memory 964, expansion memory 984, or memory on processor 952 that may be received, for example, over transceiver 968 or external interface 962.

Device 950 may communicate wirelessly through communication interface 966, which may include digital signal processing circuitry where necessary. Communication interface 966 may provide for communications under various modes or protocols, such as GSM voice calls, SMS, EMS, or MMS messaging, CDMA, TDMA, PDC, WCDMA, CDMA2000, or GPRS, among others. Such communication may occur, for example, through radio-frequency transceiver 968. In addition, short-range communication may occur, such as using a Bluetooth, WiFi, or other such transceiver (not shown). In addition, GPS (Global Positioning System) receiver module 980 may provide additional navigation- and location-related wireless data to device 950, which may be used as appropriate by applications running on device 950.

Device 950 may also communicate audibly using audio codec 960, which may receive spoken information from a user and convert it to usable digital information. Audio codec 960 may likewise generate audible sound for a user, such as through a speaker, e.g., in a handset of device 950. Such sound may include sound from voice telephone calls, may include recorded sound (e.g., voice messages, music files, etc.) and may also include sound generated by applications operating on device 950.

The computing device 950 may be implemented in a number of different forms, as shown in the figure. For example, it may be implemented as a cellular telephone 980. It may also be implemented as part of a smart phone 982, personal digital assistant, or another similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms "machine-readable medium" and "computer-readable medium" refer to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term "machine-readable signal" refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing device that includes a back end component (e.g., as a data server), or that includes a middleware component (e.g., an application server), or that includes a front end component (e.g., a client computer having a graphical user interface or a Web browser through which a user can interact with an implementation of the systems and techniques described here), or any combination of such back end, middleware, or front end components. The components of the system can be interconnected by any form or medium of digital data communication (e.g., a communication network). Examples of communication networks include a local area network ("LAN"), a wide area network ("WAN"), and the Internet.

The computing device can include clients and servers. A client and server are generally remote from each other and typically interact through a communication network. The relationship of client and server arises by virtue of computer programs running on the respective computers and having a client-server relationship to each other.

A number of embodiments have been described. Nevertheless, it will be understood that various modifications may be made without departing from the scope of the claimed invention.

In addition, the logic flows depicted in the figures do not require the particular order shown, or sequential order, to achieve desirable results. In addition, other steps may be provided, or steps may be eliminated, from the described flows, and other components may be added to, or removed from, the described systems. Accordingly, other embodiments are within the scope of the following claims.

## Claims

1. A computer-implemented method (1000) for supporting medical diagnosis of a human (10) with symptoms associated to the human's medical condition, comprising:
determining (1200), as output of a primary diagnosis model, from a set (190) of predefined medical conditions (MC1, MC2, MC3), one or more possible primary causing conditions (PCC1 to PCC3) with associated primary causing condition probabilities (PCCP1 to PCCP3) for said human, wherein medical state indicators (210 to 250) comprising said symptoms are obtained from said human and are used as inputs of the primary diagnosis model (110), and wherein a particular possible primary causing condition (PCC1) is a relevant primary causing condition that is associated with a relevant particular primary causing condition probability (PCCP1) if the associated primary causing condition probability (PCCP1) is above a predefined threshold or belongs to a predefined number of highest ranking primary causing condition probabilities;
providing (1300) the obtained medical state indicators (210 to 250) and one or more relevant primary causing conditions (PCC*) as part of a prompt (P1) to at least one pre-trained Large Language Model (121, 122, 123), used as secondary diagnosis model (120), being prompted to perform the role of a medical practitioner, wherein the secondary diagnosis model is instructed to generate an output (120o) of one or more possible secondary causing conditions (SCC1 to SCC3) with associated secondary causing condition probabilities (SCCP1 to SCCP3) for said obtained medical state indicators (210 to 250) in conformance with classes (C*) defined by the one or more relevant primary causing conditions in the prompt (P1); and
in case the generated output (120o) fails to limit the one or more possible secondary causing conditions to the classes (C*) defined by the one or more relevant primary causing conditions in the prompt (P1), reapplying (1400) said prompt (P1) to the Large Language Model until the generated output is in conformance with the classes (C*) or an abort criterion is met.

2. The method of claim 1, further comprising:
merging (1500) the output of the secondary diagnosis model (121) with the output of the primary diagnosis model (110) by:
normalizing (1510) the secondary causing condition probabilities in relation to the primary causing condition probabilities;
computing (1520) average causing condition probabilities by averaging the primary and secondary condition probabilities which relate to a same causing condition; and
outputting (1530) a consolidated causing condition list (140) with possible causing conditions (CC*) for said human (10) sorted in descending order of the associated averaged causing condition probabilities.

3. The method of any of the previous claims, wherein the prompt (P1) provides a plurality of relevant primary causing conditions to the secondary diagnosis model (120) in descending order of the associated relevant primary causing condition probabilities.

4. The method of any of the previous claims, using one or more further secondary diagnosis models (122, 123), wherein, in a parallel configuration, each secondary diagnosis model (121, 122, 123) is provided with the output of the primary diagnosis model, or, in a sequential configuration, each secondary diagnosis model (121, 122, 123) is provided with at least a subset of the outputs of the remaining secondary diagnosis models.

5. The method of any of the previous claims, wherein one or more medical state indicators are obtained by a probabilistic graphical model which uses information gain for choosing an appropriate next question in a questionnaire process applied to the human to obtain the one or more medical state indicators.

6. The method of any of the previous claims, wherein the primary diagnosis model is implemented as any of the following: a probabilistic graphical model, a decision tree, a support vector machine, a k-nearest neighbors classifier, or a logistic regression.

7. The method of any of the claims 5 to 6, wherein the obtained medical state indicators include any of free-text data, audio data, image data, or video data, and at least one of the secondary diagnosis models uses such data for generating its secondary causing conditions and associated secondary causing condition probabilities.

8. A computer program product for supporting medical diagnosis of a human (10) with symptoms associated to the human's medical condition, that when loaded into a memory of a computing device and executed by at least one processor of the computing device executes the steps of the computer-implemented method according to any of the previous claims.

9. A computer system (100) for supporting medical diagnosis of a human (10) with symptoms associated to the human's medical condition, comprising:
a primary diagnosis model (110) adapted to determine, from a set (190) of predefined medical conditions (MC1, MC2, MC3), one or more possible primary causing conditions (PCC1 to PCC3) with associated primary causing condition probabilities (PCCP1 to PCCP3) for said human, wherein medical state indicators (210 to 250) comprising said symptoms obtained from said human are used as inputs of the primary diagnosis model (110), and wherein a particular possible primary causing condition (PCC1) is a relevant primary causing condition that is associated with a relevant particular primary causing condition probability (CCP1) if the associated primary causing condition probability (CCP1) is above a predefined threshold or belongs to a predefined number of highest ranking primary causing condition probabilities; and
at least one pre-trained Large Language Model (121, 122, 123), used as secondary diagnosis model (120) and adapted for being prompted to perform the role of a medical practitioner, and to receive the obtained medical state indicators (210 to 250) and at least one or more relevant primary causing conditions (PCC*) as part of a prompt (P1), wherein the secondary diagnosis model (120) is instructed to generate an output (120o) of one or more possible secondary causing conditions (SCC1 to SCC3) with associated secondary causing condition probabilities (SCCP1 to SCCP3) for said obtained medical state indicators (210 to 250) in conformance with classes (C*) defined by the one or more relevant primary causing conditions in the prompt (P1); and
in case the generated output (120o) fails to limit the one or more possible secondary causing conditions to the classes (C*) defined by the one or more relevant primary causing conditions in the prompt (P1), the system (100) adapted to reapply said prompt (P1) to the Large Language Model until the generated output is in conformance with the classes (C*) or an abort criterion is met.

10. The system of claim 9, further comprising:
a merging module (130) adapted to merge the output of the secondary diagnosis model (120) with the output of the primary diagnosis model (110) by:
normalizing the secondary causing condition probabilities in relation to the primary causing condition probabilities;
computing average causing condition probabilities by averaging the primary and secondary condition probabilities which relate to a same causing condition; and
outputting a consolidated causing condition list (140) with possible causing conditions (CC*) for said human (10) sorted in descending order of the associated averaged causing condition probabilities.

11. The system of any of the claims 9 to 10, wherein the prompt (P1) provides relevant primary causing conditions to the secondary diagnosis model (120) in descending order of the associated relevant primary causing condition probabilities.

12. The system of any of the claims 9 to 11, using one or more further secondary diagnosis models (122, 123), wherein, in a parallel configuration, each secondary diagnosis model (121, 122, 123) is provided the output of the primary diagnosis model, or, in a sequential configuration, each secondary diagnosis model (121, 122, 123) is provided with at least a subset of the outputs of the remaining secondary diagnosis models.

13. The system of any of the claims 9 to 12, wherein one or more of the plurality of medical state indicators are obtained by a probabilistic graphical model which uses information gain for choosing an appropriate next question in a questionnaire process applied to the human to obtain the one or more medical state indicators.

14. The system of any of the claims 9 to 13, wherein the primary diagnosis model is implemented as any of the following: a probabilistic graphical model, a decision tree, a support vector machine, a k-nearest neighbors classifier, or a logistic regression.

15. The system of any of the claims 12 to 14, wherein the obtained medical state indicators include any of free-text data, audio data, image data, or video data, and at least one of the secondary diagnosis models uses such data for generating its secondary causing conditions and associated secondary causing condition probabilities.

## Patentansprüche

1. Computergestütztes Verfahren (1000) zur Unterstützung der medizinischen Diagnose eines Menschen (10) mit Symptomen, die mit dem Gesundheitszustand des Menschen in Zusammenhang stehen, umfassend:
Bestimmen (1200) als Ausgabe eines Primärdiagnosemodells aus einer Menge (190) vordefinierter medizinischer Zustände (MC1, MC2, MC3) einer oder mehrerer möglicher primärer Ursachen (PCC1 bis PCC3) mit zugehörigen Wahrscheinlichkeiten für primäre Ursachen (PCCP1 bis PCCP3) für den Menschen, wobei medizinische Zustandsindikatoren (210 bis 250) , die die Symptome umfassen, von dem Menschen erhalten und als Eingaben für das Primärdiagnosemodell (110) verwendet werden, und wobei eine bestimmte mögliche primäre Ursache (PCC1) eine relevante primäre Ursache ist, die mit einer relevanten bestimmten primären Ursachenwahrscheinlichkeit (PCCP1) verbunden ist, wenn die zugehörige primäre Ursachenwahrscheinlichkeit (PCCP1) über einem vordefinierten Schwellenwert liegt oder zu einer vordefinierten Anzahl von primären Ursachenwahrscheinlichkeiten mit der höchsten Rangfolge gehört;
Bereitstellen (1300) der erhaltenen medizinischen Zustandsindikatoren (210 bis 250) und einer oder mehrerer relevanter primärer Ursachenbedingungen (PCC*) als Teil eines Prompts (P1) an mindestens ein vortrainiertes großes Sprachmodell (121, 122, 123), das als sekundäres Diagnosemodell (120) verwendet wird und aufgefordert wird, die Rolle eines Arztes zu übernehmen, wobei das sekundäre Diagnosemodell angewiesen wird, eine Ausgabe (120o) einer oder mehrerer möglicher sekundärer Ursachenbedingungen (SCC1 bis SCC3) mit zugehörigen sekundären Ursachenerkrankungswahrscheinlichkeiten (SCCP1 bis SCCP3) für die erhaltenen medizinischen Zustandsindikatoren (210 bis 250) in Übereinstimmung mit Klassen (C*) zu generieren, die durch die eine oder mehrere relevante primäre Ursachenerkrankungen im Prompt (P1) definiert sind; und
falls die generierte Ausgabe (120o) die eine oder mehreren möglichen sekundären Ursachen nicht auf die Klassen (C*) beschränkt, die durch die eine oder mehreren relevanten primären Ursachen im Prompt (P1) definiert sind, den Prompt (P1) erneut (1400) auf das große Sprachmodell anwenden, bis die generierte Ausgabe mit den Klassen (C*) übereinstimmt oder ein Abbruchkriterium erfüllt ist.

2. Verfahren nach Anspruch 1, das ferner umfasst:
Zusammenführen (1500) der Ausgabe des Sekundärdiagnosemodells (121) mit der Ausgabe des Primärdiagnosemodells (110) durch:
Normalisieren (1510) der Wahrscheinlichkeiten der sekundären Ursachenbedingungen in Bezug auf die Wahrscheinlichkeiten der primären Ursachenbedingungen;
Berechnen (1520) der durchschnittlichen Wahrscheinlichkeiten der ursächlichen Bedingungen durch Mitteln der primären und sekundären Wahrscheinlichkeiten, die sich auf dieselbe ursächliche Bedingung beziehen; und
Ausgeben (1530) einer konsolidierten Liste der ursächlichen Bedingungen (140) mit möglichen ursächlichen Bedingungen (CC*) für den Menschen (10), sortiert in absteigender Reihenfolge der zugehörigen gemittelten Wahrscheinlichkeiten der ursächlichen Bedingungen.

3. Verfahren nach einem der vorhergehenden Ansprüche, wobei der Prompt (P1) dem sekundären Diagnosemodell (120) eine Vielzahl relevanter primärer Ursachenbedingungen in absteigender Reihenfolge der zugehörigen relevanten primären Ursachenbedingungen bereitstellt.

4. Verfahren nach einem der vorhergehenden Ansprüche unter Verwendung eines oder mehrerer weiterer sekundärer Diagnosemodelle (122, 123), wobei in einer parallelen Konfiguration jedes sekundäre Diagnosemodell (121, 122, 123) mit der Ausgabe des primären Diagnosemodells versorgt wird oder in einer sequenziellen Konfiguration jedes sekundäre Diagnosemodell (121, 122, 123) mit mindestens einer Teilmenge der Ausgaben der übrigen sekundären Diagnosemodelle versorgt wird.

5. Verfahren nach einem der vorhergehenden Ansprüche, wobei ein oder mehrere medizinische Zustandsindikatoren durch ein probabilistisches grafisches Modell erhalten werden, das Informationsgewinn verwendet, um eine geeignete nächste Frage in einem Fragebogenprozess auszuwählen, der auf den Menschen angewendet wird, um den einen oder die mehreren medizinischen Zustandsindikatoren zu erhalten.

6. Verfahren nach einem der vorhergehenden Ansprüche, wobei das Primärdiagnosemodell als eines der folgenden implementiert ist: ein probabilistisches grafisches Modell, ein Entscheidungsbaum, eine Support-Vektor-Maschine, ein k-Nearest-Neighbors-Klassifikator oder eine logistische Regression.

7. Verfahren nach einem der Ansprüche 5 bis 6, wobei die erhaltenen medizinischen Zustandsindikatoren Freitextdaten, Audiodaten, Bilddaten oder Videodaten umfassen und mindestens eines der sekundären Diagnosemodelle solche Daten zur Erzeugung seiner sekundären Ursachenbedingungen und zugehörigen sekundären Ursachenbedingungenwahrscheinlichkeiten verwendet.

8. Ein Computerprogrammprodukt zur Unterstützung der medizinischen Diagnose eines Menschen (10) mit Symptomen, die mit dem Gesundheitszustand des Menschen in Zusammenhang stehen, das, wenn es in einen Speicher eines Computergeräts geladen und von mindestens einem Prozessor des Computergeräts ausgeführt wird, die Schritte des computerimplementierten Verfahrens gemäß einem der vorherigen Ansprüche ausführt.

9. Computersystem (100) zur Unterstützung der medizinischen Diagnose eines Menschen (10) mit Symptomen, die mit dem Gesundheitszustand des Menschen in Zusammenhang stehen, umfassend:
ein Primärdiagnosemodell (110), das dazu ausgelegt ist, aus einer Menge (190) vordefinierter Gesundheitszustände (MC1, MC2, MC3) eine oder mehrere mögliche primäre Ursachen (PCC1 bis PCC3) mit zugehörigen Wahrscheinlichkeiten für primäre Ursachen (PCCP1 bis PCCP3) für den Menschen zu bestimmen, wobei medizinische Zustandsindikatoren (210 bis 250), die die von dem Menschen erhaltenen Symptome umfassen, als Eingaben für das Primärdiagnosemodell (110) verwendet werden, und wobei eine bestimmte mögliche primäre Ursache (PCC1) eine relevante primäre Ursache ist, die mit einer relevanten bestimmten primären Ursachenwahrscheinlichkeit (CCP1) verbunden ist, wenn die zugehörige primäre Ursachenwahrscheinlichkeit (CCP1) über einem vordefinierten Schwellenwert liegt oder zu einer vordefinierten Anzahl von primären Ursachenwahrscheinlichkeiten mit der höchsten Rangfolge gehört; und
mindestens ein vortrainiertes großes Sprachmodell (121, 122, 123) verwendet wird, das als sekundäres Diagnosemodell (120) verwendet wird und dafür angepasst ist, aufgefordert zu werden, die Rolle eines Arztes zu übernehmen und die erhaltenen medizinischen Zustandsindikatoren (210 bis 250) und mindestens eine oder mehrere relevante primäre Ursachenbedingungen (PCC*) als Teil eines Prompts (P1) zu empfangen, wobei das sekundäre Diagnosemodell (120) angewiesen wird, eine Ausgabe (120o) einer oder mehrerer möglicher sekundärer Ursachenbedingungen (SCC1 bis SCC3) mit zugehörigen Wahrscheinlichkeiten für sekundäre Ursachenbedingungen (SCCP1 bis SCCP3) für die erhaltenen medizinischen Zustandsindikatoren (210 bis 250) in Übereinstimmung mit Klassen (C*) zu generieren, die durch die eine oder mehreren relevanten primären Ursachenbedingungen im Prompt (P1) definiert sind; und
falls die generierte Ausgabe (120o) die eine oder mehreren möglichen sekundären Ursachenbedingungen nicht auf die Klassen (C*) beschränkt, die durch die eine oder mehreren relevanten primären Ursachenbedingungen im Prompt (P1) definiert sind, ist das System (100) so ausgelegt, dass der Prompt (P1) erneut auf das große Sprachmodell anwendet, bis die generierte Ausgabe mit den Klassen (C*) übereinstimmt oder ein Abbruchkriterium erfüllt ist.

10. Das System nach Anspruch 9, das ferner umfasst:
ein Zusammenführungsmodul (130), das so ausgelegt ist, dass es die Ausgabe des sekundären Diagnosemodells (120) mit der Ausgabe des primären Diagnosemodells (110) zusammenführt, indem es:
die Wahrscheinlichkeiten der sekundären Ursachenbedingungen in Bezug auf die Wahrscheinlichkeiten der primären Ursachenbedingungen normalisiert;
die durchschnittlichen Wahrscheinlichkeiten der Ursachenbedingungen berechnet, indem die primären und sekundären Wahrscheinlichkeiten der Bedingungen, die sich auf dieselbe Ursachenbedingung beziehen, gemittelt werden; und
eine konsolidierte Liste der Ursachenbedingungen (140) mit möglichen Ursachenbedingungen (CC*) für den Menschen (10) ausgibt, sortiert in absteigender Reihenfolge der zugehörigen gemittelten Wahrscheinlichkeiten der Ursachenbedingungen.

11. Das System nach einem der Ansprüche 9 bis 10, wobei der Prompt (P1) dem sekundären Diagnosemodell (120) relevante primäre Ursachenbedingungen in absteigender Reihenfolge der zugehörigen relevanten primären Ursachenbedingungenwahrscheinlichkeiten bereitstellt.

12. Das System nach einem der Ansprüche 9 bis 11, das ein oder mehrere weitere sekundäre Diagnosemodelle (122, 123) verwendet, wobei in einer parallelen Konfiguration jedem sekundären Diagnosemodell (121, 122, 123) die Ausgabe des primären Diagnosemodells bereitgestellt wird oder in einer sequenziellen Konfiguration jedes sekundäre Diagnosemodell (121, 122, 123) mit mindestens einer Teilmenge der Ausgaben der übrigen sekundären Diagnosemodelle versorgt wird.

13. Das System nach einem der Ansprüche 9 bis 12, wobei einer oder mehrere der mehreren medizinischen Zustandsindikatoren durch ein probabilistisches grafisches Modell erhalten werden, das Informationsgewinn verwendet, um eine geeignete nächste Frage in einem Fragebogenprozess auszuwählen, der auf den Menschen angewendet wird, um den einen oder die mehreren medizinischen Zustandsindikatoren zu erhalten.

14. Das System nach einem der Ansprüche 9 bis 13, wobei das Primärdiagnosemodell als eines der folgenden implementiert ist: ein probabilistisches grafisches Modell, ein Entscheidungsbaum, eine Support-Vektor-Maschine, ein k-Nearest-Neighbors-Klassifikator oder eine logistische Regression.

15. Das System nach einem der Ansprüche 12 bis 14, wobei die erhaltenen medizinischen Zustandsindikatoren Freitextdaten, Audiodaten, Bilddaten oder Videodaten umfassen und mindestens eines der sekundären Diagnosemodelle solche Daten verwendet, um seine sekundären Ursachenbedingungen und zugehörigen sekundären Ursachenbedingungenwahrscheinlichkeiten zu generieren.

## Revendications

1. Procédé mis en œuvre par ordinateur (1000) pour aider au diagnostic médical d'un être humain (10) présentant des symptômes associés à son état de santé, comprenant :
la détermination (1200), en tant que sortie d'un modèle de diagnostic primaire, à partir d'un ensemble (190) d'états médicaux prédéfinis (MC1, MC2, MC3), d'une ou plusieurs causes primaires possibles (PCC1 à PCC3) avec des probabilités de cause primaire associées (PCCP1 à PCCP3) pour ledit être humain, dans lequel des indicateurs d'état médical (210 à 250) comprenant lesdits symptômes sont obtenus à partir dudit être humain et sont utilisés comme entrées du modèle de diagnostic primaire (110), et dans lequel une cause primaire possible particulière (PCC1) est une cause primaire pertinente qui est associée à une probabilité de cause primaire particulière pertinente (PCCP1) si la probabilité de cause primaire associée (PCCP1) est supérieure à un seuil prédéfini ou appartient à un nombre prédéfini de probabilités de cause primaire les plus élevées ;
fournir (1300) les indicateurs d'état médical obtenus (210 à 250) et une ou plusieurs causes primaires pertinentes (PCC*) dans le cadre d'une invite (P1) à au moins un modèle linguistique de grande taille pré-entraîné (121, 122, 123), utilisé comme modèle de diagnostic secondaire (120), invité à jouer le rôle d'un praticien médical, dans lequel le modèle de diagnostic secondaire est chargé de générer une sortie (120o) d'une ou plusieurs causes secondaires possibles (SCC1 à SCC3) avec des probabilités de cause secondaire associées (SCCP1 à SCCP3) pour lesdits indicateurs d'état médical obtenus (210 à 250) conformément aux classes (C*) définies par la ou les causes primaires pertinentes dans l'invite (P1) ; et
dans le cas où le résultat généré (120o) ne parvient pas à limiter la ou les causes secondaires possibles aux classes (C*) définies par la ou les causes primaires pertinentes dans l'invite (P1), réappliquer (1400) ladite invite (P1) au modèle linguistique à grande échelle jusqu'à ce que le résultat généré soit conforme aux classes (C*) ou qu'un critère d'abandon soit rempli.

2. Procédé selon la revendication 1, comprenant en outre :
la fusion (1500) de la sortie du modèle de diagnostic secondaire (121) avec la sortie du modèle de diagnostic primaire (110) en :
normalisant (1510) les probabilités des causes secondaires par rapport aux probabilités des causes primaires ;
calculant (1520) les probabilités moyennes de cause en faisant la moyenne des probabilités de cause primaire et secondaire qui se rapportent à une même cause ; et
produisant (1530) une liste consolidée de causes (140) avec les causes possibles (CC*) pour ledit être humain (10), triées par ordre décroissant des probabilités moyennes de cause associées.

3. Procédé selon l'une quelconque des revendications précédentes, dans lequel l'invite (P1) fournit une pluralité de causes primaires pertinentes au modèle de diagnostic secondaire (120) par ordre décroissant des probabilités associées aux causes primaires pertinentes.

4. Procédé selon l'une quelconque des revendications précédentes, utilisant un ou plusieurs autres modèles de diagnostic secondaire (122, 123), dans lequel, dans une configuration parallèle, chaque modèle de diagnostic secondaire (121, 122, 123) est fourni avec la sortie du modèle de diagnostic primaire, ou, dans une configuration séquentielle, chaque modèle de diagnostic secondaire (121, 122, 123) est fourni avec au moins un sous-ensemble des sorties des modèles de diagnostic secondaire restants.

5. Procédé selon l'une quelconque des revendications précédentes, dans lequel un ou plusieurs indicateurs d'état médical sont obtenus par un modèle graphique probabiliste qui utilise le gain d'information pour choisir une question suivante appropriée dans un processus de questionnaire appliqué à l'être humain afin d'obtenir le ou les indicateurs d'état médical.

6. Procédé selon l'une quelconque des revendications précédentes, dans lequel le modèle de diagnostic primaire est mis en œuvre sous la forme de l'un quelconque des éléments suivants : un modèle graphique probabiliste, un arbre de décision, une machine à vecteurs de support, un classificateur k-plus proches voisins ou une régression logistique.

7. Procédé selon l'une quelconque des revendications 5 à 6, dans lequel les indicateurs d'état médical obtenus comprennent l'une quelconque des données de texte libre, des données audio, des données d'image ou des données vidéo, et au moins l'un des modèles de diagnostic secondaire utilise ces données pour générer ses causes secondaires et les probabilités associées aux causes secondaires.

8. Produit logiciel destiné à faciliter le diagnostic médical d'un être humain (10) présentant des symptômes associés à son état de santé, qui, lorsqu'il est chargé dans la mémoire d'un dispositif informatique et exécuté par au moins un processeur dudit dispositif, exécute les étapes de la méthode mise en œuvre par ordinateur selon l'une quelconque des revendications précédentes.

9. Système informatique (100) destiné à faciliter le diagnostic médical d'un être humain (10) présentant des symptômes associés à son état de santé, comprenant :
un modèle de diagnostic primaire (110) adapté pour déterminer, à partir d'un ensemble (190) d'états de santé prédéfinis (MC1, MC2, MC3), une ou plusieurs causes primaires possibles (PCC1 à PCC3) avec des probabilités de cause primaire associées (PCCP1 à PCCP3) pour ledit être humain, dans lequel des indicateurs d'état médical (210 à 250) comprenant lesdits symptômes obtenus à partir dudit être humain sont utilisés comme entrées du modèle de diagnostic primaire (110), et dans lequel une cause primaire possible particulière (PCC1) est une cause primaire pertinente qui est associée à une probabilité de cause primaire particulière pertinente (CCP1) si la probabilité de cause primaire associée (CCP1) est supérieure à un seuil prédéfini ou appartient à un nombre prédéfini de probabilités de causes primaires les plus élevées ; et
au moins un modèle linguistique à grande échelle pré-entraîné (121, 122, 123), utilisé comme modèle de diagnostic secondaire (120) et adapté pour être invité à jouer le rôle d'un praticien médical, et pour recevoir les indicateurs d'état médical obtenus (210 à 250) et au moins une ou plusieurs causes primaires pertinentes (PCC*) dans le cadre d'une invite (P1), dans lequel le modèle de diagnostic secondaire (120) est chargé de générer une sortie (120o) d'une ou plusieurs causes secondaires possibles (SCC1 à SCC3) avec des probabilités de causes secondaires associées (SCCP1 à SCCP3) pour lesdits indicateurs d'état médical obtenus (210 à 250) conformément aux classes (C*) définies par la ou les causes primaires pertinentes dans la demande (P1) ; et dans le cas où la sortie générée (120o) ne parvient pas à limiter la ou les causes secondaires possibles aux classes (C*) définies par la ou les causes primaires pertinentes dans l'invite (P1), le système (100) est adapté pour réappliquer ladite invite (P1) au modèle linguistique à grande échelle jusqu'à ce que la sortie générée soit conforme aux classes (C*) ou qu'un critère d'abandon soit rempli.

10. Le système selon la revendication 9, comprenant en outre :
un module de fusion (130) adapté pour fusionner la sortie du modèle de diagnostic secondaire (120) avec la sortie du modèle de diagnostic primaire (110) en :
normalisant les probabilités des causes secondaires par rapport aux probabilités des causes primaires ;
calculant les probabilités moyennes des causes en faisant la moyenne des probabilités des causes primaires et secondaires qui se rapportent à une même cause ; et
produisant une liste consolidée des causes (140) avec les causes possibles (CC*) pour ledit humain (10), triées par ordre décroissant des probabilités moyennes associées des causes.

11. Système selon l'une quelconque des revendications 9 à 10, dans lequel l'invite (P1) fournit des causes primaires pertinentes au modèle de diagnostic secondaire (120) par ordre décroissant des probabilités de causes primaires pertinentes associées.

12. Système selon l'une quelconque des revendications 9 à 11, utilisant un ou plusieurs autres modèles de diagnostic secondaire (122, 123), dans lequel, dans une configuration parallèle, chaque modèle de diagnostic secondaire (121, 122, 123) reçoit la sortie du modèle de diagnostic primaire, ou, dans une configuration séquentielle, chaque modèle de diagnostic secondaire (121, 122, 123) reçoit au moins un sous-ensemble des sorties des modèles de diagnostic secondaires restants.

13. Système selon l'une quelconque des revendications 9 à 12, dans lequel un ou plusieurs des multiples indicateurs d'état médical sont obtenus par un modèle graphique probabiliste qui utilise le gain d'information pour choisir une question suivante appropriée dans un processus de questionnaire appliqué à l'être humain afin d'obtenir le ou les indicateurs d'état médical.

14. Système selon l'une quelconque des revendications 9 à 13, dans lequel le modèle de diagnostic primaire est mis en œuvre sous la forme de l'un quelconque des éléments suivants : un modèle graphique probabiliste, un arbre de décision, une machine à vecteurs de support, un classificateur k-plus proches voisins ou une régression logistique.

15. Le système selon l'une quelconque des revendications 12 à 14, dans lequel les indicateurs d'état médical obtenus comprennent l'une quelconque des données de texte libre, des données audio, des données d'image ou des données vidéo, et au moins l'un des modèles de diagnostic secondaire utilise ces données pour générer ses causes secondaires et les probabilités associées aux causes secondaires.
